# EUROPÄISCHE PATENTSCHRIFT

(11) **EP 1 654 215 B1**
(45) Veröffentlichungstag und Bekanntmachung des Hinweises auf die Patenterteilung: **02.11.2006**
(21) Anmeldenummer: 04741043.6
(22) Anmeldetag: 15.07.2004
(51) Int. Cl.: C07C 209/64, C07C 211/14

(54) **VERFAHREN ZUR HERSTELLUNG VON ETHYLENAMINEN**
METHOD FOR PRODUCING ETHYLENE-AMINES
PROCEDE DE PRODUCTION D'ETHYLENE-AMINES

(30) Priorität: 01.08.2003 DE 10336003; 24.06.2004 DE 4030645
(43) Veröffentlichungstag der Anmeldung: 10.05.2006
(73) Patentinhaber: BASF Aktiengesellschaft, 67056 Ludwigshafen (DE)
(72) Erfinder: FRAUENKRON, Matthias, 67251 Freinsheim (DE); KRUG, Thomas, 67550 Worms (DE); EVERS, Holger, 68159 Mannheim (DE); MELDER, Johann-Peter, 67459 Böhl-Iggelheim (DE); RÖTTGER, Roderich, 68165 Mannheim (DE); SIEGERT, Markus, 69126 Heidelberg (DE); GERLACH, Till, 67071 Ludwigshafen (DE); NOUWEN, Jan, 2960 Brecht (BE); DAHLHOFF, Ellen, 67117 Limburgerhof (DE); MILLER, Christian, 67152 Ruppertsberg (DE)
(86) Internationale Anmeldenummer: PCT/EP2004/007861
(87) Internationale Veröffentlichungsnummer: WO 2005/012223

(56) Entgegenhaltungen:
- EP-A- 0 952 152
- EP-A- 1 431 273
- US-A- 5 202 490

## Beschreibung

Die vorliegende Erfindung betrifft ein Verfahren zur Herstellung von Ethylenaminen, insbesondere Diethylentriamin (DETA), Piperazin (PIP) und/oder Triethylentetramin (TETA), durch kontinuierliche Umsetzung von Ethylendiamin (EDA) in Gegenwart eines Heterogenkatalysators.

Ethylenamine finden Verwendung als Lösungsmittel, Stabilisatoren, zur Synthese von Chelat-Bildnern, Kunstharzen, Arzneimitteln, Inhibitoren und grenzflächenaktiven Substanzen.

Insbesondere Diethylentriamin (Bis(2-aminoethyl)amin; DETA) findet Verwendung als Lösungsmittel für Farbstoffe und ist Ausgangsmaterial zur Herstellung von lonenaustauschern, Schädlingsbekämpfungsmitteln, Antioxidantien, Korrosionsschutzmitteln, Komplexbildnern, Textilhilfsmitteln und Absorptionsmitteln für (saure) Gase.

Das als Edukt benötigte Ethylendiamin (H₂N-CH₂-CH₂-NH₂; EDA) kann nach bekannten Verfahren, beispielsweise durch Umsetzung von Monoethanolamin (MEOA) mit Ammoniak hergestellt werden.

Zur Herstellung von Ethylenaminen wie DETA sind in der Literatur zahlreiche Verfahren beschrieben.

Nach dem Stand der Technik werden Ethylenamine wie DETA aus Monoethanolamin (MEOA) und Ammoniak meist in Festbettreaktoren hergestellt, wobei die Katalysatoren als Aktivkomponente z.B. Nickel, Kobalt, Kupfer, Edelmetalle wie Re, Ru, Rh, Pt, Pd, oder Kombinationen davon enthalten. Trägermaterial kann beispielsweise Al₂O₃, SiO₂ oder ZrO₂ sein oder Kombinationen aus diesen und anderen Oxiden. Zur Aufrechterhaltung der Katalysatoraktivität ist es meist notwendig, geringe Mengen Wasserstoff (z.B. ca. 0,001 Gew.-% bezogen auf die Feedmenge) zuzuführen.

Als Hauptprodukt entsteht dabei EDA, als Nebenprodukte entstehen DETA, Piperazin (PIP) sowie höhere Ethylenamine, d.h. Ethylenamine mit einem Siedepunkt höher als DETA (bei gleichem Druck), und andere Verbindungen wie z.B. Aminoethylethanolamin (AEEA).
Da insbesondere DETA neben dem Hauptprodukt EDA in größeren Mengen am Markt nachgefragt wird, ist es wünschenswert, die Selektivität von DETA im Vergleich zu der in einfachem Durchgang im Festbettreaktor erhaltenen Selektivität zu erhöhen. Die Selektivität von EDA und DETA kann in gewissen Grenzen durch das molare Verhältnis von Ammoniak zu MEOA gesteuert werden. Ein hoher Ammoniak-Überschuss begünstigt, insbesondere bei geringem MEOA-Umsatz, die Bildung von EDA. Bei geringerem Ammoniak-Überschuss und größerem MEOA-Umsatz wird die Selektivität von DETA, aber auch der übrigen Nebenprodukte erhöht.

Es ist auch möglich EDA nach Aufkonzentrierung des Reaktionsaustrags teilweise in den Reaktor zurückzufahren, um die DETA-Selektivität zu erhöhen. Die Bildung der übrigen Nebenprodukte, insbesondere AEEA, kann jedoch dadurch nicht vermieden werden.

In der EP-A2-197 611 (Union Carbide Corp.) wird ein Verfahren zur Herstellung von Polyalkylenpolyaminen durch Einsatz von zwei hintereinander geschaltenen Reaktoren beschrieben.
Im ersten Reaktor erfolgt die Aminierung von MEOA mit Ammoniak an Übergangsmetallkatalysatoren (Ni, Re, Träger).
Der Reaktoraustrag wird über einen zweiten Reaktor, der ebenfalls mit einem Übergangsmetallkatalysator oder mit einem Phosphatkatalysator beladen ist, geschickt.
Zur Steuerung der Produktverteilung und Erhöhung der Selektivität bezüglich der linearen Ethylenamine wird vor dem zweiten Reaktor Ethylendiamin, das aus der Aufarbeitung des Reaktionsaustrags des zweiten Reaktors stammt und auch MEOA und H₂O enthält, zugefahren.

Nachteil dieses Verfahrens ist, dass AEEA bevorzugt zu Piperazin und nicht zu DETA weiterreagiert und durch Umsetzung von EDA mit MEOA zusätzliche Mengen an AEEA gebildet werden.

Die Synthese von DETA kann nach bekannten Verfahren auch durch Umsetzung von EDA in einem Festbettreaktor erfolgen, wobei als Nebenprodukt hauptsächlich PIP entsteht (vgl. z.B. US 5,410,086 (Burgess), GB-A-1,508,460 (BASF AG) und WO-A1-03/010125 (Akzo Nobel)) US 5,202,490 (Burgess)).
Bei einem Umsatz von z.B. etwa 30 % kann eine DETA-Selektivität von ca. 70 % erreicht werden. Bei Verwendung von reinem EDA als Edukt entsteht kein AEEA als Nebenprodukt. Die Bildung höherer Ethylenamine wird durch die Teilumsatz-Fahrweise weitgehend vermieden.
Wegen der ungünstigen Lage der chemischen Gleichgewichte würde bei höherem Umsatz jedoch vermehrt PIP gebildet werden. Wegen der Bildung von Ammoniak bei der Konvertierung von EDA zu DETA (2 EDA → DETA + NH₃) gewinnt bei höherem Umsatz außerdem zunehmend auch die Rückrektion von DETA mit Ammoniak zu EDA an Bedeutung.

Die Teilumsatz-Fahrweise führt zu hohen Kreislaufströmen an EDA (Rückführung) und damit zu einem erhöhten Energieverbrauch insbesondere in der EDA-Reinigungskolonne.

Die deutsche BASF-Patentanmeldung Nr. 10335991.5 vom 01.08.03 betrifft ein Verfahren zur Herstellung von Ethylenaminen durch Umsetzung von Monoethanolamin (ME-OA) mit Ammoniak in Gegenwart eines Katalysators in einem Reaktor (1) und Auftrennung des resultierenden Reaktionsaustrags, wobei bei der Auftrennung erhaltenes Ethylendiamin (EDA) in einem separaten Reaktor (2) in Gegenwart eines Katalysators zu Diethylentriamin (DETA) umgesetzt und der resultierende Reaktionsaustrag der Auftrennung des aus Reaktor 1 resultierenden Reaktionsaustrags zugeführt wird.

Zur Addition von Alkoholen an Olefine zu entsprechenden Ethern [z.B. MTBE (Methyltertiärbutylether) und TAME (Tertiäramylmethylether)] sind in der Literatur Verfahren bekannt, die in einer Reaktionskolonne durchgeführt werden. Die auch als Reaktivdestillation bezeichneten Verfahren sind z.B. in dem Lehrbuch 'Reactive Distillation', edited by K. Sundmacher und A. Kienle, Verlag Wiley-VCH (2003), ausführlich beschrieben.

Anwendungen der Reaktivdestillation liegen auch auf den Gebieten Veresterungen, Verseifungen und Umesterungen, Herstellung und Verseifung von Acetalen, Herstellung von Alkoholaten, Aldolkondensationen, Alkylierungen, Hydrolyse von Epoxiden, Hydratisierung von Olefinen, Isomerisierungen und Hydrierungen.

Der vorliegenden Erfindung lag die Aufgabe zugrunde, ein verbessertes wirtschaftliches Verfahren zur selektiven Herstellung von Ethylenaminen, darunter insbesondere Diethylentriamin (DETA), in hoher Ausbeute und Raum-Zeit-Ausbeute (RZA) aufzufinden.

[Raum-Zeit-Ausbeuten werden angegeben in 'Produktmenge / (Katalysatorvolumen ● Zeit)' (kg/(I_{Kat.} ● h)) und/oder 'Produktmenge / (Reaktorvolumen ● Zeit)' (kg/(I_{Reaktor} ● h)].

Demgemäß wurde ein Verfahren zur Herstellung von Ethylenaminen durch kontinuierliche Umsetzung von Ethylendiamin (EDA) in Gegenwart eines Heterogenkatalysators gefunden, welches dadurch gekennzeichnet ist, dass man die Umsetzung in einer Reaktionskolonne durchführt.

Bei den Ethylenaminen handelt es sich insbesondere um Diethylentriamin (DETA), Piperazin (PIP) und/oder Triethylentetramin (TETA).
Die Umsetzung verläuft dann z.B. nach den folgenden Gleichungen:
2 EDA → DETA + NH₃
2 EDA → PIP + 2 NH₃
3 EDA → TETA + 2 NH₃
DETA + EDA → TETA + NH₃

Erfindungsgemäß wurde erkannt, dass die Nachteile der Verfahren des Stands der Technik vermieden werden, wenn man die Synthese von Ethylenaminen, insbesondere DETA, durch kontinuierliche Umsetzung von EDA in einer Reaktionskolonne durchführt (Reaktivdestillation). Durch das kontinuierliche Abziehen von DETA und/oder TETA aus der Kolonne unterhalb der Reaktionszone (über Sumpf und/oder über einen Seitenabzug) können Folgereaktionen weitgehend unterdrückt werden und dadurch wird eine Fahrweise bei hohem Umsatz und sogar Vollumsatz von EDA ermöglicht.

Durch das kontinuierliche Entfernen von Ammoniak aus der Kolonne (bevorzugt am Kolonnenkopf, auch als Gemisch mit leichter als DETA siedenden Komponenten) wird die Rückreaktion von DETA zu EDA weitgehend unterbunden und so die Bildung von DETA beschleunigt. Die Reaktion kann daher bei anderen Drücken, vorteilhafterweise niedrigeren Drücken, durchgeführt werden, als in dem bei Verwendung eines gewöhnlichen Festbettreaktors (Rohrreaktor mit Katalysatorfestbett) optimalen Druckbereich.

Der Absolutdruck in der Kolonne liegt bevorzugt im Bereich von > 0 bis 20 bar, z.B. im Bereich von 1 bis 20 bar, insbesondere 5 bis 10 bar.

Die Temperatur in dem Bereich der Kolonne, in dem die Umsetzung von EDA zu Ethylenaminen stattfindet (Reaktionszone), liegt bevorzugt im Bereich von 100 bis 200 °C, insbesondere 140 bis 160 °C.

Die Zahl der theoretischen Trennstufen in der Kolonne insgesamt liegt bevorzugt im Bereich von 5 bis 100, besonders bevorzugt 10 bis 20.

Die Zahl der theoretischen Trennstufen in der Reaktionszone liegt bevorzugt im Bereich von 1 bis 30, insbesondere 1 bis 20, besonders 1 bis 10, z.B. 5 bis 10.

Die Zahl der theoretischen Trennstufen im Verstärkungsteil oberhalb der Reaktionszone liegt bevorzugt im Bereich von 0 bis 30, besonders 1 bis 30, weiter besonders 1 bis 15, insbesondere 1 bis 5.

Die Zahl der theoretischen Trennstufen im Abtriebsteil unterhalb der Reaktionszone liegt bevorzugt im Bereich von 0 bis 40, besonders 5 bis 30, insbesondere 10 bis 20.

Die Zugabe von EDA in die Kolonne kann unterhalb der Reaktionszone in flüssiger Form oder gasförmig erfolgen.
Die Zugabe von EDA in die Kolonne kann auch in flüssiger Form oberhalb der Reaktionszone erfolgen.

Im erfindungsgemäßen Verfahren kann sowohl reines EDA, z.B. in einer Reinheit > 98 Gew.-%, insbesondere > 99 Gew.-%, als auch EDA, weiches Piperazin (PIP), z.B. > 0 bis 25 Gew.-% PIP, und/oder andere Ethylenamine enthält, der Kolonne zugeführt werden.

Es kann auch das nach teilweiser oder vollständiger Abtrennung von Ammoniak und Wasser erhaltene EDA-Rohprodukt aus einer Umsetzung von MEOA mit Ammoniak eingesetzt werden.

Die Umsetzung wird besonders bevorzugt in Gegenwart von Wasserstoff, insbesondere in Gegenwart von 0,0001 bis 1 Gew.-%, bevorzugt 0,001 bis 0,01 Gew.-%, Wasserstoff bezogen auf die Feedmenge an EDA, durchgeführt.

Die Zugabe von Wasserstoff in die Kolonne erfolgt bevorzugt unterhalb der Reaktionszone.

Ein Gemisch aus Ammoniak, anderen Komponenten mit einem Siedepunkt tiefer als DETA (bei gleichen Druck) (Leichtsiedern) und gegebenenfalls Wasserstoff wird bevorzugt über Kopf der Kolonne entnommen.

Das über Kopf der Kolonne entnommene Gemisch kann auch Teilmengen von unumgesetztem EDA enthalten.

Das über Kopf entnommene Gemisch kann auch teilweise kondensiert und dabei Ammoniak und gegebenenfalls Wasserstoff überwiegend gasförmig entnommen (abgetrennt) und der verflüssigte Anteil kann als Rücklauf auf die Kolonne gegeben werden.

Das Gewichtsverhältnis der Rücklaufmenge der Kolonne (Kolonnenrücklaufmenge) zur Menge des Zulaufs zur Kolonne liegt dabei bevorzugt im Bereich von 0,1 bis 30, besonders bevorzugt 0,5 bis 10, insbesondere 0,5 bis 2.

Ein Gemisch aus DETA, Piperazin (PIP), TETA und anderen Komponenten mit einem Siedepunkt höher als DETA (bei gleichem Druck) (Schwersiedern) wird bevorzugt über Sumpf der Kolonne entnommen.
Das über Sumpf der Kolonne entnommene Gemisch kann auch Teilmengen von unumgesetztem EDA oder die Gesamtmenge an unumgesetzten EDA enthalten.

In einer besonderen Verfahrensausgestaltung ist die Kolonne unterhalb der Reaktionszone durch einen Seitenabzug unterteilt.
Über den Seitenabzug werden bevorzugt unumgesetztes EDA, PIP oder Mischungen davon entnommen.

Das über den Seitenabzug entnommene Produkt kann auch DETA enthalten,

Das über den Seitenabzug anfallende Produkt wird in flüssiger Form oder gasförmig entnommen.

In der Reaktionszone wird als Katalysator bevorzugt ein Katalysator enthaltend Ni, Co, Cu, Ru, Re, Rh, Pd und/oder Pt oder ein formselektiver Zeolithkatalystor oder ein Phosphatkatalysator eingesetzt.

Das oder die Metalle des Übergangsmetallkatalysators, darunter bevorzugt Ru, Re, Rh, Pd und/oder Pt, sind bevorzugt auf einem oxidischen Trägermaterial (z.B. Al₂O₃, TiO₂, ZrO₂, SiO₂) oder auf einem Zeolith oder Aktivkohle als Trägermaterial aufgebracht.

In einer bevorzugten Ausführungsform wird in der Reaktionszone als Katalysator ein Katalysator enthaltend Pd und Zirkoniumdioxid als Trägermaterial eingesetzt.

Der Gesamt-Metallgehalt der Übergangsmetall-Trägerkatalysatoren liegt bevorzugt im Bereich von > 0 bis 80 Gew.-%, besonders 0,1 bis 70 Gew.-%, weiter besonders 5 bis 60 Gew.-%, weiter besonders 10 bis 50 Gew.-%, jeweils bezogen auf das Gewicht des Trägermaterials.

Im Falle der bevorzugten Edelmetall-Trägerkatalysatoren liegt der Gesamt-Edelmetallgehalt insbesondere im Bereich von > 0 bis 20 Gew.-%, besonders 0,1 bis 10 Gew.-%, ganz besonders 0,2 bis 5 Gew.-%, weiter besonders 0,3 bis 2 Gew.-%, jeweils bezogen auf das Gewicht des Trägermaterials.

Die Heterogenkatalysatoren können in Form von festen Katalysatorbetten innerhalb der Kolonne oder in separaten Behältern außerhalb der Kolonne untergebracht werden. Sie können auch als Schüttungen, z.B. als Schüttung in eine Destillationspackung, zum Einsatz kommen, zu Füllkörpern oder Formkörpern geformt werden, beispielsweise zu Raschigringen gepresst, in Filtergewebe eingebracht und zu Rollen (sog. Bales) oder Kolonnenpackungen geformt werden, auf Destillationspackungen aufgebracht werden (Beschichtung) oder als Suspension in der Kolonne, hierbei bevorzugt als Suspension auf Kolonnenböden, eingesetzt werden.

In Verfahren mit heterogen katalysierten Reaktivdestillationen kann vorteilhaft die von der Fa. CDTech entwickelte "bales"-Technologie angewendet werden.

Weitere Technologien sind spezielle Bodenkonstruktionen mit gepackten oder suspendierten Katalysatoren.

Mehrkanalpackungen oder Kreuzkanalpackungen (siehe z.B. WO-A-03/047747) ermöglichen ein einfaches Einfüllen und Austragen von Katalysatoren, die in Teilchenform vorliegen (z.B. Kugeln, Stränglinge, Tabletten), bei geringer mechanischer Belastung des Katalysators.

Ein wichtiger Punkt bei der Reaktivdestillation ist die Bereitstellung der für den Reaktionsablauf benötigten Verweilzeit. Es ist erforderlich, die Verweilzeit der Flüssigkeit in der Kolonne im Vergleich zu einer nichtreaktiven Destillation gezielt zu erhöhen. Man nutzt Sonderkonstruktionen von Kolonneneinbauten, beispielsweise Bodenkolonnen mit Glockenböden mit stark erhöhtem Füllstand, hohe Verweilzeiten in den Ablaufschächten von Bodenkolonnen und/oder auch separat angeordnete außenliegende Verweilzeitbehälter. Anstaupackungen bieten die Möglichkeit, die Verweilzeit der Flüssigkeit um etwa den Faktor 3 gegenüber Füllkörper- und Packungskolonnen zu erhöhen.

Die Auslegung der Reaktionskolonne (z.B. Zahl der Trennstufen in den Kolonnenabschnitten Verstärkungsteil, Abtriebsteil und Reaktionszone, Rücklaufverhältnis, etc.) kann durch den Fachmann nach ihm geläufigen Methoden vorgenommen werden.

Reaktionskolonnen sind in der Literatur z:B. beschrieben in:
'Reactive distillation of nonideal multicomponent mixtures', U. Hoffmann, K. Sundmacher, March 1994, Trondheim/Norway,
'Prozesse der Reaktivdestillation', J. Stichlmair, T. Frey, Chem. Ing. Tech. 70 (1998) 12, Seiten 1507-1516,
'Thermodynamische Grundlagen der Reaktivdestillation', T. Frey, J. Stichlmair, Chem. Ing. Tech. 70 (1998) 11, Seiten 1373-1381,
WO-A-97/16243 vom 09.05.97,
DD-Patent 100701 vom 05.10.73,
US 4,267,396 vom 12.05.81,
'Reaktionen in Destillationskolonnen', G. Kaibel, H.-H. Mayer, B. Seid, Chem. Ing. Tech. 50 (1978) 8, Seiten 586-592, und dort zitierte Literatur,
DE-C2-27 14 590 vom 16.08.84,
EP-B-40724 vom 25.05.83,
EP-B-40723 vom 06.07.83,
DE-C1-37 01 268 vom 14.04.88,
DE-C1-34 13 212 vom 12.09.85,
'Production of potassium tert-butoxide by azeotropic reaction destillation', Wang Huachun, Petrochem. Eng. 26 (1997) 11,
'Design aspects for reactive distillation', J. Fair, Chem. Eng. 10 (1998), Seiten 158-162, EP-B1-461 855 vom 09.08.95,
'Consider reactive distillation', J. DeGarmo, V. Parulekar, V. Pinjala, Chem. Eng. Prog. 3 (1992),
EP-B1-402 019 vom 28.06.95,
'La distillation reaktive', P. Mikitenko, Pétrole et Techniques 329 (1986), Seiten 34-38,
'Geometry and efficiency of reactive distillation bale packing', H. Subawalla, J. González, A. Seibert, J. Fair, Ind. Eng. Chem. Res. 36 (1997), Seiten 3821-3832,
'La distillation reactive', D. Cieutat, Pétrole et Techniques 350 (1989),
'Preparation of tert-amyl alcohol in a reactive distillation column', J. González, H. Subawalla, J. Fair, Ind. Eng. Chem. Res. 36 (1997), Seiten 3845-3853,
'More uses for catalytic distillation', G. Podrebarac, G. Rempel, Chem. Tech. 5 (1997), Seiten 37-45,
'Advances in process technology through catalytic distillation', G. Gildert, K. Rock, T. McGuirk, CDTech, Seiten 103-113,
WO-A1-03/047747 vom 12.06.03 (BASF AG) und
WO-A1-97/35834.

In einer bevorzugten Ausführungsform wird das erfindungsgemäße Verfahren durchgeführt gemäß WO-A1-03/047747 in einer Kolonne zur Durchführung von Reaktivdestillationen in Gegenwart eines heterogenen teilchenförmigen Katalysators, mit einer Packung oder Füllkörpern, die im Kolonneninnenraum Zwischenräume ausbilden, wobei die Kolonne erste und zweite Teilbereiche aufweist, die alternierend angeordnet sind und sich durch die spezifische Oberfläche der Packung oder Füllkörper unterscheiden, dergestalt, dass in den ersten Teilbereichen der Quotient aus dem hydraulischen Durchmesser für den Gasstrom durch die Packung oder Füllkörper und dem äquivalenten Durchmesser der Katalysatorteilchen im Bereich von 2 bis 20, bevorzugt im Bereich von 5 bis 10 liegt, wobei die Katalysatorteilchen lose unter Einwirkung der Schwerkraft in die Zwischenräume eingebracht, verteilt und ausgetragen werden und dass in den zweiten Teilbereichen der Quotient aus dem hydraulischen Durchmesser für den Gasstrom durch die Packung oder die Füllkörper und dem äquivalenten Durchmesser der Katalysatorteilchen kleiner als 1 ist und dass in die zweiten Teilbereiche keine Katalysatorteilchen eingebracht werden. Bevorzugt wird die Kolonne hinsichtlich ihrer Gas- und/oder Flüssigkeitsbelastung so betrieben, dass maximal 50 bis 95 %, bevorzugt 70 bis 80 %, der Flutbelastung erreicht wird. Vergl. loc. cit., Ansprüche 9 und 10.

Die Aufarbeitung der im erfindungsgemäßen Verfahren anfallenden Produktströme, die vor allem das besonders gewünschte DETA, aber auch Triethylentriamin (TETA), PIP und unumgesetztes EDA enthalten, kann nach dem Fachmann bekannten Destillationsverfahren erfolgen. (Vergl. z.B. PEP Report No. 138, "Alkyl Amines", SRI International, 03/1981, Seiten 81-99, 117).

Die zur destillativen Reingewinnung der einzelnen Produkte, vor allem des besonders gewünschten DETAs, benötigten Destillationskolonnen können durch den Fachmann mit ihm geläufigen Methoden ausgelegt werden (z.B. Zahl der Trennstufen, Rücklaufverhältnis, etc.).

Die Fahrweise mit einem Seitenabzug im Abtriebsteil unterhalb der Reaktionszone der Reaktionskolonne bietet besondere Vorteile bei der weiteren Aufarbeitung zur Reingewinnung der einzelnen Produkte.

Der Seitenabzugsstrom, bestehend überwiegend aus PIP, unumgesetztem EDA oder Mischungen davon, enthält nur kleine Mengen an DETA und Schwersiedern, insbesondere im Falle der gasförmigen Entnahme des Seitenabzugsstroms. Er kann daher im Aufarbeitungsteil, getrennt vom Sumpfabzugsstrom der Reaktionskolonne, direkt an der Stelle zugeführt werden, wo die Reinigung von EDA und PIP durchgeführt wird, anstatt erst die Abtrennung der leichtsiedenden Komponenten von DETA und Schwersiedern zu durchlaufen.

Teilmengen des Seitenstroms können auch in die Reaktionskolonne selbst zurückgeführt werden. Dies ist insbesondere vorteilhaft, wenn der Seitenstrom vorwiegend EDA und wenig oder kein PIP enthält.

Der Sumpfabzugsstrom der Reaktionskolonne enthält bei dieser Fahrweise weniger Leichtsieder (EDA und PIP), so dass die Kolonne zur Abtrennung der leichtsiedenden Komponenten von DETA und Schwersiedern entlastet wird.

Wenn die Reaktivdestillation bei kleinen Drücken durchgeführt wird, beispielsweise 1 bis 3 bar, ist es auch möglich, den Sumpfabzugsstrom bei Sumpftemperaturen von etwa 200 bis 240 °C frei von EDA und PIP zu erhalten. Der Sumpfabzugsstrom kann dann optional im Aufarbeitungsteil an der Stelle zugeführt werden, wo die Reinigung von DETA durchgeführt wird, anstatt erst die Abtrennung der leichtsiedenden Komponenten von DETA und Schwersiedern zu durchlaufen.

Das erfindungsgemäße Verfahren ermöglicht die Herstellung von DETA mit einer Selektivität von > 18 %, insbesondere > 20 %, ganz besonders > 22 %, jeweils bezogen auf EDA, bei einem EDA-Umsatz von > 30 %, insbesondere > 40 %, ganz besonders > 50%.

### Beispiele

### Beispiel A

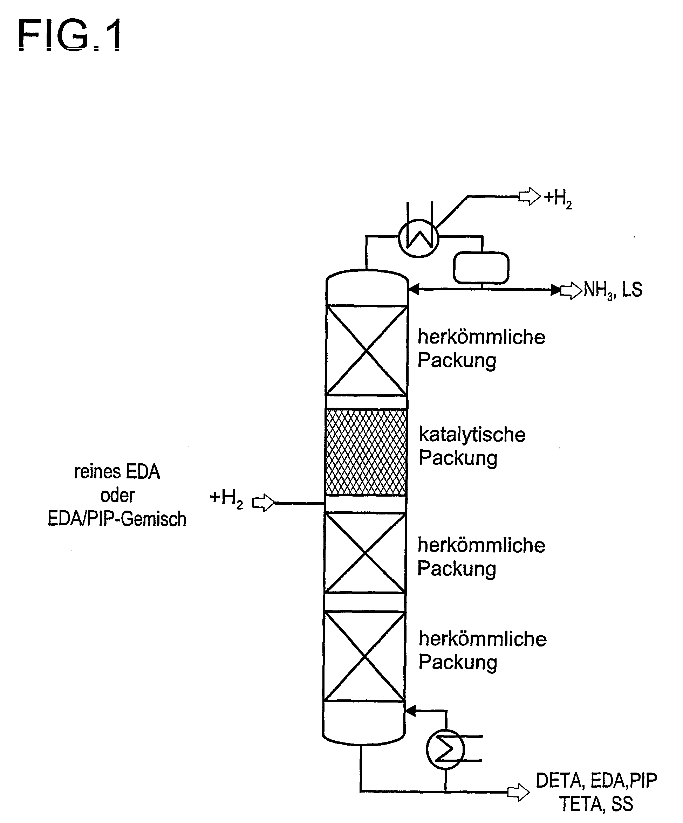

Abbildung 1 in Anlage 1 zeigt eine Ausgestaltung des erfindungsgemäßen Verfahrens bei dem reines EDA oder ein EDA/PIP-Gemisch zusammen mit Wasserstoff der Reaktionskolonne kontinuierlich unterhalb der katalytischen Packung zugeführt wird und ein Gemisch enthaltend DETA, unumgesetztes EDA, PIP, TETA und Schwersieder (SS, d.h. Komponenten mit einem Siedepunkt höher als DETA) über Sumpf erhalten wird. Ammoniak, Wasserstoff und Leichtsieder (LS, d.h. Komponenten mit einem Siedepunkt tiefer als DETA) werden über Kopf abgetrennt.

### Beispiel B

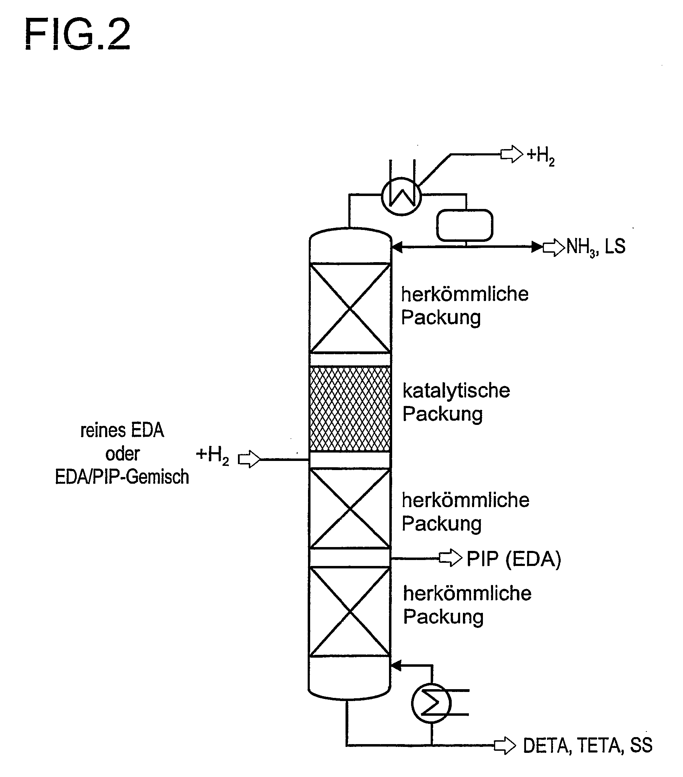

Abbildung 2 in Anlage 2 zeigt eine Ausgestaltung des erfindungsgemäßen Verfahrens bei dem reines EDA oder ein EDA/PIP-Gemisch zusammen mit Wasserstoff der Reaktionskolonne kontinuierlich unterhalb der katalytischen Packung zugeführt wird und ein Gemisch enthaltend DETA, TETA und Schwersieder (SS, d.h. Komponenten mit einem Siedepunkt höher als DETA) über Sumpf erhalten wird. Ammoniak, Wasserstoff und Leichtsieder (LS, d.h. Komponenten mit einem Siedepunkt tiefer als DETA) werden über Kopf abgetrennt.
In einem Seitenabzug im Abtriebsteil unterhalb der Reaktionszone der Reaktionskolonne wird PIP, optional als Gemisch mit EDA, abgetrennt.

### Beispiele 1 bis 4

Für die Herstellung des Katalysators wurden Zirkoniumdioxid-Stränge mit einem Durchmesser von 3,2 mm und einer Länge von 1 - 2 cm verwendet.
2700 g des Trägers wurden getränkt mit 770,0 ml (= 92 % der H₂O-Aufnahme) einer wässrigen Palladiumnitratlösung, so dass eine berechnete Palladium-Beladung von 0,9 Gew.-% Palladium resultierte. Die Tränkung wurde mehrfach durchgeführt. Danach wurde 6 h bei 120°C (aufgeheizt auf 120°C innerhalb 1 h) im Trockenschrank getrocknet und 2 h bei 450°C (aufgeheizt auf 450°C innerhalb 2 h) im Muffelofen kalziniert.

Alle Umsetzungen der Beispiele 1 bis 4 wurden bei einem Absolutdruck von 5 bar durchgeführt. Es wurden 6 l/h Wasserstoff unterhalb der katalytischen Schicht in die Kolonne eingespeist.

Für die Beispiele 1 und 2 wurden von dem hergestellten Katalysator 755 g in eine Laborkoionne mit 55 mm Durchmesser und einer Packung, wie in der Patentanmeldung WO-A1-03//047747, Anspruch 9 und dort zugehöriger Beschreibung, ausgeführt, eingefüllt.
Die Zahl der theoretischen Stufen unterhalb der Katalysatorschüttung betrug 6. Die Katalysatorpackung hat eine theoretische Stufenzahl von 3,5. Oberhalb der Katalysatorschüttung betrug die Zahl der theoretischen Stufen 1.

### Beispiel 1

Es wurden 400 g/h EDA flüssig bei Raumtemperatur oberhalb der katalytischen Schicht zugefahren. Der Rücklauf wurde auf 800 g/h eingestellt. Im stationären Zustand stellte sich eine Sumpftemperatur von 186°C ein.

Der Sumpfaustrag der Kolonne wies eine Zusammensetzung (in Gew.-%) von 65 % EDA, 9,7 % DETA und 16 % Piperazin auf. Die übrigen Komponenten waren Hochsieder.
Damit erhielt man bei einem EDA-Umsatz von 41 % eine Selektivität für DETA von 25 %.

### Beispiel 2

Es wurden 400 g/h EDA flüssig bei Raumtemperatur unterhalb der katalytischen Schicht zugefahren. Der Rücklauf wurde auf 400 g/h eingestellt. Im stationären Zustand stellte sich eine Sumpftemperatur von 183°C ein.
Der Sumpfaustrag der Kolonne wies eine Zusammensetzung (in Gew.-%) von 74,6 % EDA, 6,1 % DETA und 13,2 % Piperazin auf. Die übrigen Komponenten waren Hochsieder.
Damit erhielt man bei einem EDA-Umsatz von 30,6% eine Selektivität für DETA von 21,7 %.

Für das Beispiel 3 wurden von dem hergestellten Katalysator 934 g in eine Laborkolonne mit 55 mm Durchmesser und einer Packung, wie in der Patentanmeldung WO-A1-03//047747, Anspruch 9 und dort zugehöriger Beschreibung, ausgeführt, eingefüllt.

Die Zahl der theoretischen Stufen unterhalb der Katalysatorschüttung betrug 15. Die Katalysatorpackung hat eine theoretische Stufenzahl von 10. Oberhalb der Katalysatorschüttung betrug die Zahl der theoretischen Stufen 10.

### Beispiel 3

Es wurden 100 g/h EDA flüssig bei Raumtemperatur oberhalb der katalytischen Schicht zugefahren. Der Rücklauf wurde auf 800 g/h eingestellt. Im stationären Zustand stellte sich eine Sumpftemperatur von 162°C ein.

Der Sumpfaustrag der Kolonne wies eine Zusammensetzung (in Gew.-%) von 55 % EDA, 12 % DETA und 21 % Piperazin auf. Die übrigen Komponenten waren Hochsieder.
Damit erhielt man bei einem EDA-Umsatz von 55 % eine Selektivität für DETA von 21 %.

## Patentansprüche

1. Verfahren zur Herstellung von Ethylensminen durch kontinuierliche Umsetzung von Ethylendiamin (EDA) in Gegenwart eines Heterogenkatalysators, **dadurch gekennzeichnet, dass** man die Umsetzung in einer Reaktionskolonne mittels Reaktivdestillation durchführt.

2. Verfahren zur Herstellung von Ethylenaminen nach Anspruch 1, wobei es sich bei den Ethylenaminen um Diethylentriamin (DETA), Piperazin (PIP) und/oder Triethylentetramin (TETA) handelt.

3. Verfahren nach den Ansprüchen 1 oder 2, **dadurch gekennzeichnet, dass** der Absolutdruck in der Kolonne im Bereich von > 0 bis 20 bar liegt.

4. Verfahren nach einem der vorhergehenden Ansprüche, **dadurch gekennzeichnet, dass** die Temperatur in dem Bereich der Kolonne, in dem die Umsetzung von EDA zu Ethylenaminen stattfindet (Reaktionszone), im Bereich von 100 bis 200 °C liegt.

5. Verfahren nach einem der vorhergehenden Ansprüche, **dadurch gekennzeichnet, dass** die Zahl der theoretischen Trennstufen in der Kolonne insgesamt im Bereich von 5 bis 100 liegt.

6. Verfahren nach einem der vorhergehenden Ansprüche, **dadurch gekennzeichnet, dass** die Zahl der theoretischen Trennstufen in der Reaktionszone im Bereich von 1 bis 30 liegt.

7. Verfahren nach einem der vorhergehenden Ansprüche, **dadurch gekennzeichnet, dass** die Zahl der theoretischen Trennstufen im Verstärkungsteil oberhalb der Reaktionszone im Bereich von 0 bis 30 liegt.

8. Verfahren nach einem der vorhergehenden Ansprüche, **dadurch gekennzeichnet, dass** die Zahl der theoretischen Trennstufen im Abtriebsteil unterhalb der Reaktionszone im Bereich von 0 bis 40 liegt.

9. Verfahren nach einem der vorhergehenden Ansprüche, **dadurch gekennzeichnet, dass** in der Reaktionszone als Katalysator ein Katalysator enthaltend Ni, Co, Cu, Ru, Re, Rh, Pd und/oder Pt oder ein formselektiver Zeolithkatalystor oder ein Phosphatkatalysator eingesetzt wird.

10. Verfahren nach einem der vorhergehenden Ansprüche, **dadurch gekennzeichnet, dass** in der Reaktionszone als Katalysator ein Katalysator enthaltend Pd und Zirkoniumdioxid als Trägermaterial eingesetzt wird.

11. Verfahren nach einem der vorhergehenden Ansprüche, **dadurch gekennzeichnet, dass** der Katalysator als Schüttung in die Reaktionskolonne eingebracht ist.

12. Verfahren nach einem der vorhergehenden Ansprüche, **dadurch gekennzeichnet, dass** der Katalysator als Schüttung in eine Destillationspackung eingebracht ist.

13. Verfahren nach einem der Ansprüche 1 bis 10, **dadurch gekennzeichnet, dass** der Katalysator als Beschichtung auf einer Destillationspackung vorliegt.

14. Verfahren nach einem der Ansprüche 1 bis 10, **dadurch gekennzeichnet, dass** der Katalysator in einem außerhalb der Kolonne befindlichen Verweilzeitbehälter vorliegt.

15. Verfahren nach einem der vorhergehenden Ansprüche, **dadurch gekennzeichnet, dass** die Zugabe von EDA in die Kolonne in flüssiger Form unterhalb der Reaktionszone erfolgt.

16. Verfahren nach einem der Ansprüche 1 bis 14, **dadurch gekennzeichnet, dass** die Zugabe von EDA in die Kolonne gasförmig unterhalb der Reaktionszone erfolgt.

17. Verfahren nach einem der Ansprüche 1 bis 14, **dadurch gekennzeichnet, dass** die Zugabe von EDA in die Kolonne in flüssiger Form oberhalb der Reaktionszone erfolgt.

18. Verfahren nach einem der vorhergehenden Ansprüche, **dadurch gekennzeichnet, dass** das EDA der Kolonne in einer Reinheit > 98 Gew.-% zugeführt wird.

19. Verfahren nach einem der vorhergehenden Ansprüche, **dadurch gekennzeichnet, dass** das der Kolonne zugeführte EDA Piperazin (PIP) und/oder andere Ethylenamine enthält.

20. Verfahren nach einem der vorhergehenden Ansprüche, **dadurch gekennzeichnet, dass** die Umsetzung in Gegenwart von Wasserstoff durchgeführt wird.

21. Verfahren nach dem vorhergehenden Anspruch, **dadurch gekennzeichnet, dass** die Umsetzung in Gegenwart von 0,0001 bis 1 Gew.-% Wasserstoff bezogen auf die Feedmenge an EDA durchgeführt wird.

22. Verfahren nach einem der beiden vorhergehenden Ansprüche, **dadurch gekennzeichnet, dass** die Zugabe von Wasserstoff in die Kolonne unterhalb der Reaktionszone erfolgt.

23. Verfahren nach einem der vorhergehenden Ansprüche, **dadurch gekennzeichnet, dass** über Kopf der Kolonne ein Gemisch aus Ammoniak, anderen Komponenten mit einem Siedepunkt tiefer als DETA (Leichtsiedern) und gegebenenfalls Wasserstoff entnommen wird.

24. Verfahren nach dem vorhergehenden Anspruch, **dadurch gekennzeichnet, dass** das über Kopf der Kolonne entnommene Gemisch auch Teilmengen von unumgesetztem EDA enthält.

25. Verfahren nach einem der beiden vorhergehenden Ansprüche, **dadurch gekennzeichnet, dass** das über Kopf entnommene Gemisch teilweise kondensiert wird und dabei Ammoniak und gegebenenfalls Wasserstoff überwiegend gasförmig entnommen werden und der verflüssigte Anteil als Rücklauf auf die Kolonne gegeben wird.

26. Verfahren nach einem der vorhergehenden Ansprüche, **dadurch gekennzeichnet, dass** das Gewichtsverhältnis der Rücklaufmenge der Kolonne zur Menge des Zulaufs zur Kolonne im Bereich von 0,1 bis 30 liegt.

27. Verfahren nach einem der vorhergehenden Ansprüche, **dadurch gekennzeichnet, dass** über Sumpf der Kolonne ein Gemisch aus DETA, Piperazin (PIP), TETA und anderen Komponenten mit einem Siedepunkt höher als DETA (Schwersiedem) entnommen wird.

28. Verfahren nach dem vorhergehenden Anspruch, **dadurch gekennzeichnet, dass** das über Sumpf der Kolonne entnommene Gemisch auch Teilmengen von unumgesetztem EDA oder die Gesamtmenge an unumgesetzten EDA enthält.

29. Verfahren nach einem der vorhergehenden Ansprüche, **dadurch gekennzeichnet, dass** die Kolonne unterhalb der Reaktionszone durch einen Seitenabzug unterteilt ist.

30. Verfahren nach dem vorhergehenden Anspruch, **dadurch gekennzeichnet, dass** über den Seitenabzug unumgesetztes EDA, PIP oder Mischungen davon entnommen werden.

31. Verfahren nach einem der beiden vorhergehenden Ansprüche, **dadurch gekennzeichnet, dass** über den Seitenabzug entnommenes Produkt DETA enthält.

32. Verfahren nach einem der drei vorhergehenden Ansprüche, **dadurch gekennzeichnet, dass** über den Seitenabzug anfallendes Produkt in flüssiger Form entnommen wird.

33. Verfahren nach einem der Ansprüche 29 bis 31, **dadurch gekennzeichnet, dass** über den Seitenabzug anfallendes Produkt gasförmig entnommen wird.

34. Verfahren nach einem der vorhergehenden Ansprüche zur Herstellung von DETA mit einer Selektivität von > 20 %, bezogen auf EDA, bei einem EDA-Umsatz von > 30 %.

## Claims

1. A process for the preparation of ethyleneamines by continuous reaction of ethylenediamine (EDA) in the presence of a heterogeneous catalyst, which comprises carrying out the reaction in a reaction column by means of reactive distillation.

2. The process for the preparation of ethyleneamines according to claim 1, where the ethyleneamines are diethylenetriamine (DETA), piperazine (PIP) and/or triethylenetetramine (TETA).

3. The process according to claims 1 or 2, wherein the absolute pressure in the column is in the range from > 0 to 20 bar.

4. The process according to any of the preceding claims, wherein the temperature in the section of the column in which the reaction of EDA to ethyleneamines takes place (reaction zone) is in the range from 100 to 200°C.

5. The process according to any of the preceding claims, wherein the number of theoretical plates in the column is in the range from 5 to 100 in total.

6. The process according to any of the preceding claims, wherein the number of theoretical plates in the reaction zone is in the range from 1 to 30.

7. The process according to any of the preceding claims, wherein the number of theoretical plates in the enriching section above the reaction zone is in the range from 0 to 30.

8. The process according to any of the preceding claims, wherein the number of theoretical plates in the stripping section below the reaction zone is in the range from 0 to 40.

9. The process according to any of the preceding claims, wherein the catalyst used in the reaction zone is a catalyst comprising Ni, Co, Cu, Ru, Re, Rh, Pd and/or Pt or a shape-selective zeolite catalyst or a phosphate catalyst.

10. The process according to any of the preceding claims, wherein the catalyst used in the reaction zone is a catalyst comprising Pd and zirconium dioxide support material.

11. The process according to any of the preceding claims, wherein the catalyst is introduced into the reaction column in the form of a loose bed.

12. The process according to any of the preceding claims, wherein the catalyst is introduced into a distillation packing in the form of a loose bed.

13. The process according to any of claims 1 to 10, wherein the catalyst is in the form of a coating on a distillation packing.

14. The process according to any of claims 1 to 10, wherein the catalyst is in a retention container situated above the column.

15. The process according to any of the preceding claims, wherein the addition of EDA to the column takes place in liquid form below the reaction zone.

16. The process according to any of claims 1 to 14, wherein the addition of EDA to the column takes place in gaseous form below the reaction zone.

17. The process according to any of claims 1 to 14, wherein the addition of EDA to the column takes place in liquid form above the reaction zone.

18. The process according to any of the preceding claims, wherein EDA is passed to the column in a purity of > 98% by weight.

19. The process according to any of the preceding claims, wherein the column comprises introduced EDA, piperazine (PIP) and/or other ethyleneamines.

20. The process according to any of the preceding claims, wherein the reaction is carried out in the presence of hydrogen.

21. The process according to the preceding claim, wherein the reaction is carried out in the presence of from 0.0001 to 1% by weight of hydrogen, based on the feed amount of EDA.

22. The process according to either of the two preceding claims, wherein the addition of hydrogen to the column takes place below the reaction zone.

23. The process according to any of the preceding claims, wherein a mixture of ammonia, other components with a boiling point lower than DETA (low-boiling components) and if appropriate hydrogen is removed via the top of the column.

24. The process according to the preceding claim, wherein the mixture removed from the top of the column also comprises partial amounts of unreacted EDA.

25. The process according to either of the two preceding claims, wherein the mixture removed overhead is partially condensed, and during this ammonia and if appropriate hydrogen are removed predominantly in gaseous form, and the liquefied fraction is fed to the column as reflux.

26. The process according to any of the preceding claims, wherein the weight ratio of the amount of reflux in the column to the amount of feed to the column is in the range from 0.1 to 30.

27. The process according to any of the preceding claims, wherein a mixture of DETA, piperazine (PIP), TETA and other components with a boiling point higher than DETA (high-boiling components) is removed by the bottom of the column.

28. The process according to the preceding claim, wherein the mixture removed by the bottom of the column also comprises partial amounts of unreacted EDA or the total amount of unreacted EDA.

29. The process according to any of the preceding claims, wherein the column below the reaction zone is divided by a side take-off.

30. The process according to the preceding claim, wherein unreacted EDA, PIP or mixtures thereof are removed via the side take-off.

31. The process according to any of the preceding claims, wherein product removed via the side take-off comprises DETA.

32. The process according to any of the three preceding claims, wherein product produced via the side take-off is removed in liquid form.

33. The process according to any of claims 29 to 31, wherein product produced via the side take-off is removed in gaseous form.

34. The process according to any of the preceding claims for producing DETA at a selectivity of > 20%, based on EDA, coupled with an EDA conversion of > 30%.

## Revendications

1. Procédé de fabrication d'éthylèneamines par conversion continue d'éthylènediamine (EDA) en présence d'un catalyseur hétérogène, **caractérisé en ce que** l'on effectue la conversion dans une colonne de réaction au moyen d'une distillation réactive.

2. Procédé de fabrication d'éthylèneamines selon la revendication 1, dans lequel les éthylèneamines sont la diéthylènetriamine (DETA), la pipérazine (PIP) et/ou la triéthylènetétramine (TETA).

3. Procédé selon les revendications 1 ou 2, **caractérisé en ce que** la pression absolue dans la colonne se situe dans une plage de plus de 0 à 20 bars.

4. Procédé selon l'une quelconque des revendications précédentes, **caractérisé en ce que** la température dans la zone de la colonne, dans laquelle se fait la conversion de l'EDA en éthylèneamines (zone réactionnelle), se situe dans une plage de 100 à 200°C.

5. Procédé selon l'une quelconque des revendications précédentes, **caractérisé en ce que** le nombre des étapes de séparation théoriques dans la colonne se situe, au total, dans une plage de 5 à 100.

6. Procédé selon l'une quelconque des revendications précédentes, **caractérisé en ce que** le nombre des étapes de séparation théoriques dans la zone réactionnelle se situe dans une plage de 1 à 30.

7. Procédé selon l'une quelconque des revendications précédentes, **caractérisé en ce que** le nombre des étapes de séparation théoriques dans la partie de renforcement, se trouvant au-dessus de la zone réactionnelle, se situe dans une plage de 0 à 30.

8. Procédé selon l'une quelconque des revendications précédentes, **caractérisé en ce que** le nombre des étapes de séparation théoriques dans la partie de soutirage, se trouvant en dessous de la zone réactionnelle, se situe dans une plage de 0 à 40.

9. Procédé selon l'une quelconque des revendications précédentes, **caractérisé en ce que** l'on emploie, comme catalyseur dans la zone réactionnelle, un catalyseur contenant les éléments Ni, Co, Cu, Ru, Re, Rh, Pd et/ou Pt, ou un catalyseur de zéolite de forme sélective ou un catalyseur de phosphate.

10. Procédé selon l'une quelconque des revendications précédentes, **caractérisé en ce que** l'on utilise dans la zone réactionnelle un catalyseur contenant du Pd comme catalyseur et du dioxyde de zirconium comme matériau de support.

11. Procédé selon l'une quelconque des revendications précédentes, **caractérisé en ce que** le catalyseur est introduit en vrac dans la colonne réactionnelle.

12. Procédé selon l'une quelconque des revendications précédentes, **caractérisé en ce que** le catalyseur est introduit en vrac dans un garnissage de distillation.

13. Procédé selon l'une quelconque des revendications 1 à 10, **caractérisé en ce que** le catalyseur se présente sous la forme d'une couche sur un garnissage de distillation.

14. Procédé selon l'une quelconque des revendications 1 à 10, **caractérisé en ce que** le catalyseur se trouve dans un récipient de séjour situé en dehors de la colonne.

15. Procédé selon l'une quelconque des revendications précédentes, **caractérisé en ce que** l'on effectue l'addition de l'EDA dans la colonne sous forme liquide en dessous de la zone réactionnelle.

16. Procédé selon l'une quelconque des revendications 1 à 14, **caractérisé en ce que** l'on effectue l'addition de l'EDA dans la colonne sous forme gazeuse en dessous de la zone réactionnelle.

17. Procédé selon l'une quelconque des revendications 1 à 14, **caractérisé en ce que** l'on effectue l'addition de l'EDA dans la colonne sous forme liquide au-dessus de la zone réactionnelle.

18. Procédé selon l'une quelconque des revendications précédentes, **caractérisé en ce que** l'on délivre à la colonne l'EDA en pureté supérieure à 98% en poids.

19. Procédé selon l'une quelconque des revendications précédentes, **caractérisé en ce que** l'EDA délivrée à la colonne contient de la pipérazine (PIP) et/ou d'autres éthylèneamines.

20. Procédé selon l'une quelconque des revendications précédentes, **caractérisé en ce que** la conversion est effectuée en présence d'hydrogène.

21. Procédé selon la revendication précédente, **caractérisé en ce que** la conversion est effectuée en présence de 0,0001 à 1% en poids d'hydrogène par rapport à la quantité d'EDA acheminée.

22. Procédé selon l'une quelconque des deux revendications précédentes, **caractérisé en ce que** l'on effectue l'addition d'hydrogène dans la colonne en dessous de la zone réactionnelle.

23. Procédé selon l'une quelconque des revendications précédentes, **caractérisé en ce que** l'on prélève en tête de colonne un mélange constitué d'ammoniac, d'autres composants ayant un point d'ébullition inférieur à celui de la DETA (substances à bas point d'ébullition) et, éventuellement, d'hydrogène.

24. Procédé selon la revendication précédente, **caractérisé en ce que** le mélange prélevé en tête de colonne contient également des quantités partielles d'EDA n'ayant pas réagi.

25. Procédé selon l'une quelconque des deux revendications précédentes, **caractérisé en ce que** le mélange prélevé en tête de colonne est partiellement condensé, **en ce que** l'on prélève, en l'occurrence, de l'ammoniac et éventuellement de l'hydrogène principalement sous forme gazeuse et **en ce que** la fraction liquéfiée est recyclée à la colonne.

26. Procédé selon l'une quelconque des revendications précédentes, **caractérisé en ce que** le rapport pondéral de la quantité recyclée à la colonne à la quantité délivrée à la colonne se situe dans la plage de 0,1 à 30.

27. Procédé selon l'une quelconque des revendications précédentes, **caractérisé en ce que** l'on prélève en bas de colonne un mélange constitué de DETA, de pipérazine (PIP), de TETA et d'autres composants ayant un point d'ébullition supérieur à celui de la DETA (substances à haut point d'ébullition).

28. Procédé selon la revendication précédente, **caractérisé en ce que** le mélange, prélevé en bas de colonne, contient également des quantités partielles d'EDA n'ayant pas réagi ou contient la totalité de la quantité d'EDA n'ayant pas réagi.

29. Procédé selon l'une quelconque des revendications précédentes, **caractérisé en ce que** la colonne est sub-divisée, en dessous de la zone réactionnelle, par un soutirage latéral.

30. Procédé selon la revendication précédente, **caractérisé en ce que** l'on prélève par le soutirage latéral de l'EDA n'ayant pas réagi, de la PIP ou des mélanges de celle-ci.

31. Procédé selon l'une des deux revendications précédentes, **caractérisé en ce que** le produit prélevé par le soutirage latéral contient de la DETA.

32. Procédé selon l'une des trois revendications précédentes, **caractérisé en ce que** le produit obtenu par le soutirage latéral est prélevé sous forme liquide.

33. Procédé selon l'une quelconque des revendications 29 à 31, **caractérisé en ce que** le produit obtenu par le soutirage latéral est prélevé sous forme gazeuse.

34. Procédé selon l'une quelconque des revendications précédentes, destiné à la fabrication de DETA avec une sélectivité supérieure à 20% par rapport à l'EDA, dans le cas d'une conversion d'EDA supérieure à 30%.
